# EUROPEAN PATENT APPLICATION

(11) **EP 4 535 375 A1**
(43) Date of publication of application: **09.04.2025**
(21) Application number: 23815545.1
(22) Date of filing: 28.03.2023
(51) Int. Cl.: H01B 1/16, A61B 5/263, H01B 13/00, H01G 11/22, H01G 11/86, H01M 4/136, H01M 4/1397

(54) **ELECTRODE AND METHOD FOR MANUFACTURING ELECTRODE**

(30) Priority: 01.06.2022 JP 2022089861
(71) Applicant: Murata Manufacturing Co., Ltd., Nagaokakyo-shi, Kyoto 617-8555 (JP)
(72) Inventor: HIRAYAMA, Mayu, Nagaokakyo-shi, Kyoto 617-8555 (JP); FUJIWAKI, Mika, Nagaokakyo-shi, Kyoto 617-8555 (JP); SHIMAZAKI, Toshiko, Nagaokakyo-shi, Kyoto 617-8555 (JP); TORITA, Takeshi, Nagaokakyo-shi, Kyoto 617-8555 (JP)
(74) Representative: Frick, Robert
(86) International application number: PCT/JP2023/012598
(87) International publication number: WO 2023/233783

(57) **Abstract**

The present disclosure is to provide an electrode having low impedance, and preferably an electrode having low surface impedance. An electrode comprising a film comprising two-dimensional particles,
wherein the two-dimensional particles comprise at least a metal cation and one or a plurality of layers,
the layer comprises:
a layer body represented by a formula:

MₘXₙ,

wherein M is at least one Group 3, 4, 5, 6, or 7 metal and comprises at least a Ti atom, X is a carbon atom, a nitrogen atom, or a combination thereof, n is 1 or more and 4 or less, m is more than n and 5 or less; and
a modifier or terminal T presents on a surface of the layer body, wherein T is at least one selected from the group consisting of a hydroxyl group, a fluorine atom, a chlorine atom, an oxygen atom, and a hydrogen atom,
wherein the metal cation comprises a Li cation, and
wherein a content of the Li cation in the two-dimensional particles is 5.4 mol or more with respect to 100 mol of the Ti atom.

## Description

### TECHNICAL FIELD

The present disclosure relates to an electrode and a method for manufacturing an electrode.

### BACKGROUND ART

In recent years, MXene has been attracting attention as a new material having conductivity. MXene is a type of so-called two-dimensional material, and as will be described later, is a layered material in the form of one or plural layers. In general, MXene is in the form of particles (which comprises powders, flakes, nanosheets, and the like) of such a layered material.

Currently, various studies are conducted toward the application of MXene to various electrical devices. For example, Patent Documents 1 and 2 describe that MXene in the form of a film can be used as an electrode for nerve signal measurements (Patent Document 1).

### PRIOR ART DOCUMENT

### NON-PATENT DOCUMENT

Non-patent Document 1: Nicolette Driscoll, et al., "Two-Dimensional Ti3C2 MXene for High Resolution Neural Interfaces," ACS Nano, 2018, Vol. 12, Issue 10, pp. 10419-10429
Non-patent Document 2: Nicolette Driscoll, et al., "MXene-infused bioelectronic interfaces for multiscale electrophysiology and stimulation," Science Translational Medicine, 2021, Vol. 13, Issue 612, article abf8629

### SUMMARY

### PROBLEMS TO BE SOLVED

The present disclosure is to provide an electrode having low impedance, and preferably an electrode having low surface impedance. The present disclosure is also to provide a method for manufacturing the electrode.

### SOLUTIONS TO THE PROBLEMS

An electrode of the present disclosure comprises a film comprising two-dimensional particles,
wherein the two-dimensional particles comprise
at least a metal cation and one or a plurality of layers,
the layer comprises:
   a layer body represented by a formula:

      MₘXₙ,
   wherein M is at least one Group 3, 4, 5, 6, or 7 metal and comprises at least a Ti atom, X is a carbon atom, a nitrogen atom, or a combination thereof, n is 1 or more and 4 or less,
      m is more than n and 5 or less; and
   a modifier or terminal T present on a surface of the layer body, wherein at least one selected from the group consisting of a hydroxyl group, a fluorine atom, a chlorine atom, an oxygen atom, and a hydrogen atom,
   wherein the metal cation comprises a Li cation, and
   wherein a content of the Li cation in the two-dimensional particles is 5.4 mol or more with respect to 100 mol of the Ti atom.

A method for manufacturing an electrode of the present disclosure, the method comprising forming a film using two-dimensional particles,
wherein the two-dimensional particles comprise
at least an intercalated product manufactured by a manufacturing method comprising:
   (a) providing a precursor represented by a formula:

      MₘAXₙ

      wherein M is at least one Group 3, 4, 5, 6, or 7 metal and comprises at least a Ti atom, X is a carbon atom, a nitrogen atom, or a combination thereof, A is at least one Group 12, 13, 14, 15, or 16 element, n is 1 or more and 4 or less, and m is more than n and 5 or less;
   (b) removing at least a part of A atoms from the precursor using an etching solution to obtain an etched product;
   (c) cleaning the etched product to obtain an etched cleaned product; and
   (d) mixing the etched cleaned product with a metal compound comprising a metal cation to obtain an intercalated product in which the etched cleaned product is intercalated with the metal cation,
wherein the metal cation comprises a Li cation.

### EFFECTS OF THE INVENTION

According to the present disclosure, an electrode having low impedance can be provided, and preferably an electrode having low surface impedance can be provided. The present disclosure can also provide a method for manufacturing the electrode.

### BRIEF DESCRIPTION OF THE DRAWINGS

[Fig. 1] Figs. 1(a) and 1(b) are schematic cross-sectional views showing MXene particles as a layered material according to one embodiment of the present disclosure, in which Fig. 1(a) shows single-layer MXene particles, and Fig. 1(b) shows multilayer (exemplarily, two-layer) MXene particles.

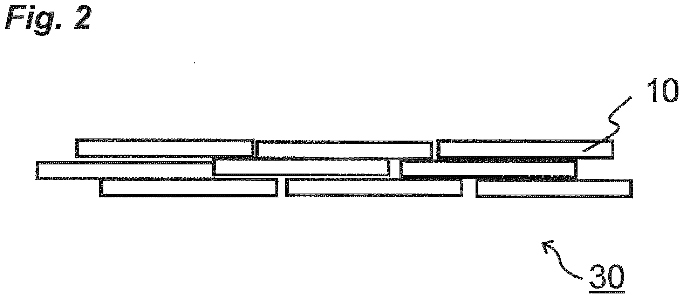

[Fig. 2] Fig. 2 is a schematic cross-sectional view showing a conductive film according to one embodiment of the present disclosure.

### DETAILED DESCRIPTION

Hereinafter, an electrode and a method for manufacturing the electrode according to one embodiment of the present disclosure will be described.

An electrode of the present disclosure comprises a film comprising two-dimensional particles,
wherein the two-dimensional particles comprise at least a metal cation and one or a plurality of layers,
the layer comprises:
   a layer body represented by the following formula,
   wherein M is at least one Group 3, 4, 5, 6, or 7 metal and comprises at least a Ti atom, X is a carbon atom, a nitrogen atom, or a combination thereof, n is 1 or more and 4 or less, m is more than n and 5 or less; and
   a modifier or terminal T present on a surface of the layer body, wherein at least one selected from the group consisting of a hydroxyl group, a fluorine atom, a chlorine atom, an oxygen atom, and a hydrogen atom,
   wherein the metal cation comprises a Li cation, and
   wherein a content of the Li cation is 5.4 mol or more with respect to 100 mol of the Ti atom.

The electrode of the present disclosure has the above configuration, and exhibits low impedance. Although it should not be construed as being limited to a specific theory, since the two-dimensional particles comprised in the film are a layered material and comprise a certain amount of Li cations, charge transfer may occur more smoothly, and the conductivity of the two-dimensional particles may become high. Therefore, the electrode comprises the film may suppress the impedance low, particularly the surface impedance.

The two-dimensional particles may be understood as a layered material or layered compound, and is also represented as "MₘXₙTₛ", wherein s is any number, and conventionally x or z may be used instead of s. Typically, n can be 1, 2, 3, or 4, but is not limited thereto.

In the present disclosure, the layer may be referred to as an MXene layer, and the two-dimensional particles may be referred to as MXene two-dimensional particles or MXene particles.

In the above formula of MXene, it is preferable that M is at least one selected from the group consisting of Ti, Zr, Hf, V, Nb, Ta, Cr, Mo, and Mn, and comprises at least Ti, and it is more preferable that M is at least one selected from the group consisting of Ti, V, Cr, and Mo, and comprises at least Ti.

The ratio of the Ti atom in M may be preferably 50 atom% to 100 atom%, more preferably 70 atom% to 100 atom%, and still more preferably 90 atom% to 100 atom%.

MXenes whose above formula MₘXₙ is expressed as below are known:
Sc₂C, Ti₂C, Ti₂N, Zr₂C, Zr₂N, Hf₂C, Hf₂N, V₂C, V₂N, Nb₂C, Ta₂C, Cr₂C, Cr₂N, Mo₂C, Mo_{1.3}C, Cr_{1.3}C, (Ti,V)₂C, (Ti,Nb)₂C, W₂C, W_{1.3}C, Mo₂N, Nb_{1.3}C, Mo_{1.3}Y₀₆C (In the above formula, "1.3" and "0.6" mean about 1.3 (= 4/3) and about 0.6 (= 2/3), respectively.),
Ti₃C₂, Ti₃N₂, Ti₃(CN), Zr₃C₂, (Ti,V)₃C₂, (Ti₂Nb)C₂, (Ti₂Ta)C₂, (Ti₂Mn)C₂, Hf₃C₂, (Hf₂V)C₂, (Hf₂Mn)C₂, (V₂Ti)C₂, (Cr₂Ti)C₂, (Cr₂V)C₂, (Cr₂Nb)C₂, (Cr₂Ta)C₂, (Mo₂Sc)C₂, (Mo₂Ti)C₂, (Mo₂Zr)C₂, (Mo₂Hf)C₂, (Mo₂V)C₂, (Mo₂Nb)C₂, (Mo₂Ta)C₂, (W₂Ti)C₂, (W₂Zr)C₂, (W₂Hf)C₂,
Ti₄N₃, V₄C₃, Nb₄C₃, Ta₄C₃, (Ti,Nb)₄C₃, (Nb,Zr)₄C₃, (Ti₂Nb₂)C₃, (Ti₂Ta₂)C₃, (V₂Ti₂)C₃, (V₂Nb₂)C₃, (V₂Ta₂)C₃, (Nb₂Ta₂)C₃, (Cr₂Ti₂)C₃, (Cr₂V₂)C₃, (Cr₂Nb₂)C₃, (Cr₂Ta₂)C₃, (Mo₂Ti₂)C₃, (Mo₂Zr₂)C₃, (Mo₂Hf₂)C₃, (Mo₂V₂)C₃, (Mo₂Nb₂)C₃, (Mo₂Ta₂)C₃, (W₂Ti₂)C₃, (W₂Zr₂)C₃, (W₂Hf₂)C₃, (Mo_{2.7}V_{1.3})C₃ (In the above formula, "2.7" and "1.3" mean about 2.7 (= 8/3) and about 1.3 (= 4/3), respectively.)

Typically, in the above formula, M may comprise Ti and X may be a carbon atom or a nitrogen atom. Preferably, M may be Ti and X may be a carbon atom. For example, the MAX phase is Ti₃AlC₂ and MXene is Ti₃C₂Tₛ (in other words, M is Ti, X is C, n is 2, and m is 3).

In the present disclosure, MXene may comprise a relatively small amount of A atoms derived from the MAX phase of the precursor, for example, 10% by mass or less with respect to the original amount of the A atoms. The remaining amount of A atoms can be preferably 8% by mass or less, and more preferably 6% by mass or less. However, even if the remaining amount of the A atoms exceeds 10% by mass, there may be no problem depending on the application and use conditions of the two-dimensional particles.

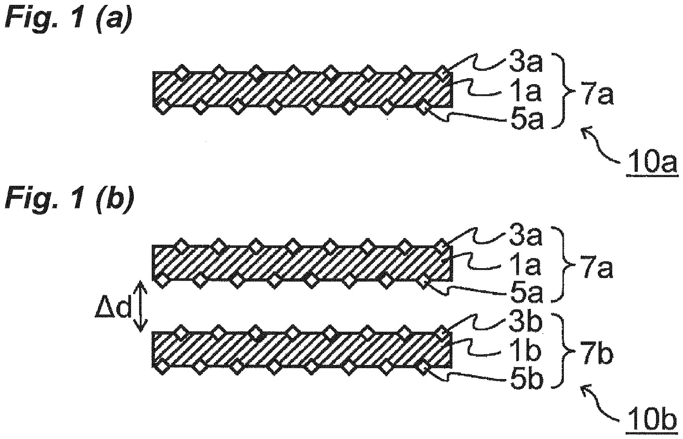

The two-dimensional particles are an aggregate comprising MXene particles (hereinafter, simply referred to as "MXene particles")10a (single-layer MXene particles) as one layer schematically exemplified in Fig. 1(a). More specifically, the MXene particles 10a are an MXene layer 7a comprising a layer body (MₘXₙ layer) 1a represented by MₘXₙ and modifiers or terminals T3a and 5a present on the surface (more specifically, at least one of two surfaces facing each other in each layer) of the layer body 1a. Therefore, the MXene layer 7a is also represented by "MₘXₙTₛ" wherein s is any number.

The two-dimensional particles may comprise one or a plurality of layers. Examples of the MXene particles (multilayer MXene particles) comprising the plurality of layers comprise, but are not limited to, MXene particles 10b comprising two layers as schematically shown in Fig. 1(b). 1b, 3b, 5b, and 7b in Fig. 1(b) are the same as 1a, 3a, 5a, and 7a in Fig. 1(a) described above. Two adjacent MXene layers (for example, 7a and 7b) of the multilayer MXene particles may not necessarily be completely separated from each other, but may be partially in contact with each other. The MXene particles 10a may be a mixture of the single-layer MXene particles 10a and the multilayer MXene particles 10b, in which the multilayer MXene particles 10b are individually separated and present as one layer and the multilayer MXene particles 10b that are not separated remain.

Although the present embodiment is not limited, the thickness of each layer (corresponding to the MXene layers 7a and 7b) comprised in the MXene particles may be, for example, 0.8 nm to 5 nm, and particularly 0.8 nm to 3 nm (may mainly vary depending on the number of M atomic layers comprised in each layer). For individual laminates of the multilayer MXene particles that may be comprised, an interlayer distance (alternatively, a void dimension, indicated by Δd in Fig. 1(b)) may be, for example, 0.8 nm to 10 nm, particularly 0.8 nm to 5 nm, and more particularly about 1 nm, and the total number of layers may be 2 to 20,000.

In one aspect, the ratio of (the average value of the major axes of the two-dimensional surfaces of the two-dimensional particles)/(the average value of the thicknesses of the two-dimensional particles) is 1.2 or more, preferably 1.5 or more, and more preferably 2 or more. The average value of the major axes of the two-dimensional surfaces of the two-dimensional particles and the average value of the thicknesses of the two-dimensional particles may be obtained by a method described later.

The two-dimensional particles of the present embodiment preferably comprise MXene particles having a large number of layers. The term "large number of layers" means that, for example, the number of laminated MXene layers is 7 or more. The thickness of the MXene particles having a large number of layers in the lamination direction is preferably more than 15 nm, more preferably 18 nm to50 nm, and still more preferably 18 nm to 30 nm. In the MXene particles having a large number of layers, the ratio of (the average value of the major axes of the two-dimensional surfaces of the two-dimensional particles)/(the average value of the thicknesses of the two-dimensional particles) is, for example, more than 5 and 50 or less, preferably 10 to 30, and more preferably 11 to 20. This can make it easy to comprise a large amount of metal cations when the two-dimensional particles comprise the MXene particles having a large number of layers.

Examples of the MXene particles having a large number of layers comprise two-dimensional particles obtained without performing delamination treatment.

The two-dimensional particles of the present embodiment preferably comprise multilayer MXene particles having a small number of layers. The term "small number of layers" means that, for example, the number of laminated MXene layers is 6 or less. The thickness of the multilayer MXene particles having a small number of layers in the lamination direction is preferably 15 nm or less, and more preferably 10 nm or less. In the multilayer MXene particles having a small number of layers, the ratio of (the average value of the major axes of the two-dimensional surfaces of the two-dimensional particles)/(the average value of the thicknesses of the two-dimensional particles) is 1.2 or more, preferably 1.5 to 10, and more preferably 2 to 5. Hereinafter, the "MXene particles having a small number of layers" may be referred to as "few-layer MXene particles". The single-layer MXene particles and the few-layer MXene particles may be collectively referred to as "single-layer/few-layer MXene particles". As a result, the film formability of the film comprising the two-dimensional particles can be improved.

Examples of the single-layer/few-layer MXene particles comprise two-dimensional particles obtained through delamination treatment.

In one aspect, the two-dimensional particles of the present embodiment preferably comprise the MXene particles having a large number of layers and the single-layer/few-layer MXene particles. The proportion of the MXene particles having a large number of layers in the two-dimensional particles of the present embodiment can be preferably 20 vol% to 100 vol%, more preferably 30 vol% to 100 vol%, and still more preferably 60 vol% to100 vol%. This can make it easy to obtain two-dimensional particles comprising a large amount of metal cations.

In one aspect, the two-dimensional particles of the present embodiment preferably comprise single-layer MXene particles and few-layer MXene particles, that is, single-layer/few-layer MXene particles. The proportion of the single-layer/few-layer MXene particles having a thickness of 15 nm or less in the two-dimensional particles of the present embodiment can be preferably 0 vol% to 70 vol%, more preferably 0 vol% to 60 vol%, and still more preferably 0 vol% to 25 vol%. As a result, the film formability of the film comprising the two-dimensional particles can be improved.

### (Average Value of Major Axes of Two-Dimensional Surfaces of Two-Dimensional Particles)

In the two-dimensional particles of the present embodiment, the average value of the major axes of the two-dimensional surfaces is preferably 1 µm to 20 µm. Hereinafter, the average value of the major axes of the two-dimensional surfaces may be referred to as "average flake size".

As the average flake size is larger, the orientation of the two-dimensional particles in a material comprising the two-dimensional particles is better. The orientation of the two-dimensional particles can be evaluated by, for example, the conductivity of the material comprising the two-dimensional particles.

The average value of the major axes of the two-dimensional surfaces is preferably 1.5 µm or more, and more preferably 2.5 µm or more. When the delamination treatment of MXene is performed by subjecting MXene to ultrasonic treatment, most of MXene is reduced in diameter to about several hundred nanometers in terms of major axis by the ultrasonic treatment, so that the film formed of the single-layer MXene delaminated by the ultrasonic treatment is considered to have low orientation of the two-dimensional particles.

The average value of the major axes of the two-dimensional surfaces is, for example, 20 µm or less, preferably 15 µm or less, and more preferably 10 µm or less from the viewpoint of dispersibility in a dispersion medium.

As shown in Examples described later, the major axis of the two-dimensional surface refers to a major axis when each MXene particle is approximated to an elliptical shape in an electron micrograph, and the average value of the major axes of the two-dimensional surfaces refers to the number average of the major axes of 80 particles or more. As an electron microscope, a scanning electron microscope (SEM) photograph or a transmission electron microscope (TEM) photograph can be used.

The average value of the major axes of the two-dimensional particles of the present embodiment may be measured in a state where a material comprising the two-dimensional particles is dissolved in a solvent and the two-dimensional particles are dispersed in the solvent. Alternatively, the average value of the major axes of the two-dimensional particles may be measured from the SEM image of the material.

### (Average Value of Thicknesses of Two-Dimensional Particles)

The average value of the thicknesses of the two-dimensional particles of the present embodiment is preferably 1 nm to 15 nm. The thickness is preferably 10 nm or less, more preferably 7 nm or less, and still more preferably 5 nm or less. Meanwhile, considering the thickness of the single-layer MXene particles, the lower limit of the thickness of the two-dimensional particles can be 1 nm.

The average value of the thicknesses of the two-dimensional particles is obtained as a number average dimension (for example, a number average of at least 40 particles) based on an atomic force microscope (AFM) photograph or a transmission electron microscope (TEM) photograph.

The two-dimensional particles preferably comprise voids. The inclusion of the voids in the two-dimensional particles makes it easier to comprise a large amount of metal cations, and can make it easier to obtain an electrode having high conductivity and low impedance.

The metal cation comprises a monovalent Li cation, and may further comprise another metal cation.

However, the metal of the metal cation is different from the M atom. The metal of the metal cation is different from A atoms comprised in a precursor described later.

In one aspect, the content of the Li cation in the two-dimensional particles may be 5.4 mol or more, preferably 5.4 mol to 67 mol, more preferably 5.4 mol to20 mol, and still more preferably 5.4 mol to9.7 mol, with respect to 100 mol of the Ti atom. As a result, the conductivity of the film comprising the two-dimensional particles is enhanced, which may results in an electrode having low impedance.

The content of a Li atom in the two-dimensional particles may be, for example, 0.1% by mass to 20% by mass, further 0.1% by mass to10% by mass, especially 0.2% by mass to and 3% by mass, and particularly 0.2% by mass to0.5% by mass. This may further enhance the conductivity of the film comprising the two-dimensional particles, and can make it easier to obtain an electrode having low impedance.

The contents of the Ti atom, Li atom, metal cation, and Li cation in the two-dimensional particles can be measured by, for example, inductively coupled plasma atomic emission spectrometry (ICP-AES) or the like.

The content of the Li cation in the metal cation may be, for example, 1 mol% to 100 mol%, preferably 70 mol% to 100 mol%, more preferably 80 mol% to100 mol%, and still more preferably 90 mol% to100 mol%.

The metal cation is typically present on the layer. That is, the metal cation may be in contact with the layer, or may be present on the layer via another element.

In one aspect, the two-dimensional particles preferably comprise a first component having a large number of layers and a Li cation content of 5.4 mol or more with respect to 100 mol of the Ti atom. By comprising the two-dimensional particles of the first component, it is possible to easily form a film comprising a large amount of metal cations while maintaining film formability.

The thickness of the two-dimensional particles of the first component may be preferably more than 15 nm, more preferably 18 nm to 50 nm, and still more preferably 18 nm to 30 nm, and the number of layers may be preferably more than 5 and 50 or less, preferably more than 10 and 30 or less, and more preferably 11 to 20. In the two-dimensional particles of the first component, the content of the Li cation can be preferably 5.4 mol to 69 mol, more preferably 5.4 mol to 9.7 mol, and still more preferably 6 mol to 9.7 mol, with respect to 100 mol of the Ti atom. Furthermore, the two-dimensional particles of the first component preferably comprise voids.

The content of the first component comprised in the two-dimensional particles may be preferably 20 vol% to 100 vol%, more preferably 30 vol% to 100 vol%, and still more preferably 60 vol% to 100 vol%. This may results in the formation of a film comprising a large amount of metal cations while maintaining film formability.

Typically, the first component can be manufactured as an intercalated product described later, but is not limited to those manufactured by the manufacturing method.

In one aspect, the two-dimensional particles preferably comprise a second component having a small number of layers and a Li cation content of more than 0 mol and less than 5.4 mol with respect to 100 mol of the Ti atom. As a result, the film formability of the film comprising the two-dimensional particles can be improved.

The thickness of the two-dimensional particles of the second component may be preferably 15 nm or less, and more preferably 10 nm or less, and the number of layers may be preferably 1 to 6. In the two-dimensional particles of the second component, the content of the Li cation may be preferably 1 mol or more and less than 5.4 mol, and particularly 5.3 mol or less, with respect to 100 mol of the Ti atom.

In the two-dimensional particles, the content of the second component may be preferably 0 vol% to 70 vol%, more preferably 0 vol% to 60 vol%, and still more preferably 0 vol% to 25 vol% with respect to 100 parts by volume of the first component.

The second component can be typically manufactured as a delaminated product described later, but is not limited to those manufactured by the manufacturing method.

In the electrode of the present disclosure, the content of the two-dimensional particles comprised in the film may be preferably 70 vol% to 100 vol%, more preferably 80 vol% to 100 vol%, and still more preferably 90 vol% to 100 vol%. This may result in an electrode having high conductivity and low impedance.

Hereinafter, a method for manufacturing two-dimensional particles will be described in detail, but the present disclosure is not limited to the embodiment.

The method for manufacturing two-dimensional particles, the method comprises
(a) providing a precursor represented by the following formula:

   MₘAXₙ

   wherein M is at least one Group 3, 4, 5, 6, or 7 metal and comprises at least a Ti atom, X is a carbon atom, a nitrogen atom, or a combination thereof, A is at least one Group 12, 13, 14, 15, or 16 element, n is 1 or more and 4 or less, and m is more than n and 5 or less; and
(b) removing at least a part of A atoms from the precursor using an etching solution to obtain an etched product. The method may comprise
(c) cleaning the etched product to obtain an etched cleaned product, and
(d) mixing the etched cleaned product with a metal compound comprising a metal cation to obtain an intercalated product in which the etched cleaned product is intercalated with the metal cation,
(e) stirring the intercalated product to obtain a delaminated product in which the intercalated product is delaminated, and
(f) cleaning the delaminated product to obtain a delaminated cleaned product. wherein the metal cation in the step (d) comprises a Li cation.

In the present disclosure, any of the etched product, the etched cleaned product, the intercalated product, the delaminated product, and the delaminated cleaned product can be comprised in the technical scope of the two-dimensional particles.

Hereinafter, each step will be described in detail.

### · Step (a)

First, a predetermined precursor is prepared. The predetermined precursor that can be used in the present embodiment is a MAX phase that is a precursor of MXene, and is represented by the following formula:

MₘAXₙ

wherein M is at least one Group 3, 4, 5, 6, or 7 metal and comprises at least Ti, X is a carbon atom, a nitrogen atom, or a combination thereof, A is at least one Group 12, 13, 14, 15, or 16 element, n is 1 or more and 4 or less, m is more than n and 5 or less.

The above M, X, n, and m are as described above.

A is at least one Group 12, 13, 14, 15, and 16 element, is usually a Group A element, typically Group IIIA and Group IVA, and more specifically may comprise at least one selected from the group consisting of Al, Ga, In, Tl, Si, Ge, Sn, Pb, P, As, S, and Cd, and is preferably Al.

The MAX phase has a crystal structure in which a layer constituted by the A atoms is located between two layers represented by MₘXₓ (each X may have a crystal lattice located in an octahedral array of M). When typically m = n + 1, but not limited thereto, the MAX phase comprises repeating units in which each one layer of X atoms is disposed in between adjacent layers of n + 1 layers of M atoms (these are also collectively referred to as an "MₘXₙ layer"), and a layer of A atoms ("A atom layer") is disposed as a layer next to the (n + 1)th layer of M atoms. The A atom layer (and optionally a part of the M atoms) is removed by selectively etching (removing and optionally layer-separating) the A atoms (and optionally a part of the M atoms) from the MAX phase.

The MAX phase can be manufactured by a known method. For example, TiC powder, Ti powder, and Al powder are mixed in a ball mill, and the resulting mixed powder is calcined under an Ar atmosphere to obtain a calcined body (block-shaped MAX phase). Thereafter, the obtained calcined body can be pulverized by an end mill to obtain a powdery MAX phase for the next step.

### · Step (b)

In the step (b), an etching treatment for removing at least a part of the A atoms from MₘAXₙ of the precursor by etching is performed using an etching solution. As a result, a treated product in which at least a part of the layer composed of A atoms is removed is obtained while the layer represented by MₘXₙ in the precursor is maintained.

The etching solution may comprise an acid such as HF, HCl, HBr, HI, sulfuric acid, phosphoric acid, or nitric acid, and typically, an etching solution comprising F atoms can be used. Examples of the etching solution comprise a mixed liquid of LiF and hydrochloric acid; a mixed liquid of hydrofluoric acid and hydrochloric acid; and a mixed liquid comprising hydrofluoric acid, and these mixed liquids may further comprise phosphoric acid or the like. The etching solution can be typically an aqueous solution.

As the etching operation using the etching solution and other conditions, conventional conditions can be adopted.

### · Step (c)

In the step (c), the treated product obtained by the etching treatment is cleaned to obtain an etched cleaned product. By performing cleaning, the acid and the like used in the etching treatment can be sufficiently removed.

The cleaning can be performed using a cleaning liquid, and typically, can be performed by mixing the etched product and the cleaning liquid. The cleaning liquid typically comprises water, and preferably pure water. Meanwhile, a small amount of hydrochloric acid or the like may be further comprised in addition to the pure water. The amount of the cleaning liquid to be mixed with the etched product and the method for mixing the etched product and the cleaning liquid are not particularly limited. For example, as the mixing method, stirring, centrifugation, and the like are performed in a state where the etched product and the cleaning liquid coexist. Examples of the stirring method comprise a stirring method using a handshake, an automatic shaker, a share mixer, a pot mill, or the like. The degree of stirring such as a stirring speed or a stirring time may be adjusted according to the amount, concentration, and the like of the etched product to be treated. The cleaning with the cleaning liquid may be performed once or more, and the cleaning with the cleaning is preferably performed a plurality of times. For example, specifically, the cleaning with the cleaning liquid may be performed by sequentially performing step (i) (to the treated product or the remaining precipitate obtained in the following (iii)), adding the cleaning liquid, followed by stirring, step (ii) centrifuging the stirred product, and step (iii) discarding a supernatant after centrifugation, and the steps (i) to (iii) are repeated within a range of 2 times or more, for example, 15 times or less.

### · Step (d)

In the step (d), an intercalation treatment of intercalating a metal cation is performed on the etched cleaned product using a metal compound comprising a metal cation to obtain an intercalated product. As a result, an intercalated product in which the metal cation is intercalated between two adjacent MₘXₙ layers is obtained. The intercalation treatment may be performed in a dispersion medium.

The metal cation may be the same as the metal cation comprised in the two-dimensional particles, and may comprise a Li cation and another metal cation.

The metal of the metal cation is different from the M atom. The metal of the metal cation is different from the A atoms comprised in the precursor.

Examples of the metal compound comprise an ionic compound in which the metal cation and the anion are bonded. Examples of the ionic compound comprise an iodide, a phosphate, a sulfate comprising a sulfide salt, a nitrate, an acetate, and a carboxylate of the metal cation. As the metal cation, a lithium ion is preferable, and as the metal compound, a metal compound comprising a lithium ion is preferable, an ionic compound of a lithium ion is more preferable, and one or more of an iodide, a phosphate, and a sulfide salt of a lithium ion are still more preferable. The usage of a lithium ion is considered to assist the formation of a monolayer due to the fact that that water hydrated to the lithium ion has the most negative dielectric constant.

The specific method of the intercalation treatment is not particularly limited, and for example, the etched cleaned product and the metal compound may be mixed and stirred, or may be left to stand. For example, stirring at room temperature can be exemplified. Examples of the stirring method comprise a method using a stirring bar such as a stirrer, a method using a stirring blade, a method using a mixer, and a method using a centrifugal device. The stirring time can be set according to the production scale of the single-layer/few-layer MXene particles, and can be set, for example, to 12 to 24 hours.

The intercalation treatment may be performed in the presence of a dispersion medium. Examples of the dispersion medium comprise water; and organic media such as N-methylpyrrolidone, N-methylformamide, N,N-dimethylformamide, methanol, ethanol, dimethylsulfoxide, ethylene glycol, and acetic acid.

The order of mixing the dispersion medium, the etched cleaned product, and the metal compound is not particularly limited, but in one aspect, the dispersion medium and the etched cleaned product may be mixed, followed by mixing the mixture with the metal compound. Typically, the etching solution after the etching treatment may be used as the dispersion medium.

The intercalation treatment can be typically performed on the etched cleaned product, but in another aspect, the intercalation treatment may be performed on the precursor simultaneously with the etching treatment. Specifically, the etching and intercalation treatments comprise mixing a precursor, an etching solution, and a metal compound comprising a metal cation to remove at least a part of A atoms from the precursor, and intercalating the precursor with the metal cation from which the A atoms have been removed to obtain an intercalated product. As a result, at least a part of the A atoms are removed from the precursor (MAX), the MₘXₙ layer in the precursor remains, and an intercalated product in which the metal cation is intercalated between the plurality of adjacent MₘXₙ layers is obtained.

As the etching solution and the metal compound used in the etching and intercalation treatments, the same etching solution and metal compound as those used in the step (b) can be used, respectively.

The intercalated product may be cleaned before being subjected to the production of an electrode to form an intercalated cleaned product, and the intercalated cleaned product is also comprised in the range of the intercalated product. The excess metal compound can be removed by cleaning the intercalated product.

The intercalated product can be cleaned using a cleaning liquid, and typically, can be cleaned by mixing the intercalated product and the cleaning liquid. As the cleaning liquid, the same cleaning liquid as that in the step (c) can be used, and the mixing can be performed by the same method as the mixing method in the step (c). For example, specifically, water can be used as the cleaning liquid. The cleaning with the cleaning liquid may be performed by sequentially performing step (i) (to the treated product or the remaining precipitate obtained in the following (iii)), adding the cleaning liquid, followed by stirring, step (ii) centrifuging the stirred product, and step (iii) discarding a supernatant after centrifugation, and the steps (i) to (iii) are repeated within a range of 2 times or more, for example, 15 times or less.

### · Step (e)

In the step (e), the intercalated product is stirred, and a delamination treatment for delaminating the intercalated product is performed to obtain a delaminated product. By the stirring, a shear stress is applied to the intercalated product, and at least a part between two adjacent MₘXₙ layers can be peeled off, and the MXene particles can be formed into a single-layer or a few-layer.

Conditions for the delamination treatment are not particularly limited, and the delamination treatment can be performed by a known method. Examples of a method for applying a shear stress to the intercalated product comprise a method for dispersing the intercalated product in a dispersion medium and stirring the dispersion medium. Examples of the stirring method comprise stirring using a mechanical shaker, a vortex mixer, a homogenizer, ultrasonic treatment, a handshake, an automatic shaker, or the like. The degree of stirring such as a stirring speed and a stirring time may be adjusted according to the amount, concentration, and the like of the treated product to be treated. For example, the slurry after the intercalation is centrifuged to discard the supernatant, and then pure water is added to the remaining precipitate, followed by stirring by, for example, a handshake or an automatic shaker to perform layer separation. The removal of the unpeeled substance comprises a step of performing centrifugal separation to discard the supernatant, and then cleaning the remaining precipitate with water. For example, (i) pure water is added to the remaining precipitate after discarding the supernatant, followed by stirring, (ii) centrifugation is performed, and (iii) the supernatant is recovered. This operation of (i) to (iii) is repeated 1 time or more, preferably 2to 10 times to obtain a supernatant comprising single-layer/few-layer MXene particles as a delaminated product. Alternatively, the supernatant may be centrifuged, and the supernatant after centrifugation may be discarded to obtain a clay comprising single-layer/few-layer MXene particles as the delaminated product.

### · Step (f)

In the step (f), the delaminated product is cleaned to obtain a delaminated cleaned product. By the cleaning, impurities and the like can be removed.

In one aspect, the cleaning can be performed using a cleaning liquid, and typically, can be performed by mixing the delaminated product and the cleaning liquid. In another aspect, the cleaning can be performed by acid-treating the delaminated product and then mixing the acid-treated product with the cleaning liquid. As the acid, inorganic acids such as hydrochloric acid, sulfuric acid, nitric acid, phosphoric acid, perchloric acid, hydroiodic acid, hydrobromic acid, and hydrofluoric acid; and organic acids such as acetic acid, citric acid, oxalic acid, benzoic acid, and sorbic acid may be appropriately used, and the concentration of the acid in the acid solution can be appropriately adjusted according to the delaminated product. The cleaning with the cleaning liquid may be performed by sequentially performing step (i) (to the treated product or the remaining precipitate obtained in the following (iii)), adding the cleaning liquid, followed by stirring, step (ii) centrifuging the stirred product, and step (iii) discarding a supernatant after centrifugation, and the steps (i) to (iii) are repeated within a range of 2 times or more, for example, 15 times or less. The stirring can be performed using a handshake, an automatic shaker, a share mixer, a pot mill, or the like. The acid treatment may be performed one or more times, and if necessary, an operation of mixing with a fresh acid solution (acid solution not used for the acid treatment) and stirring may be performed within a range of two or more times, for example, 10 times or less. As the cleaning liquid, the same cleaning liquid as that in the step (c) can be used, and the mixing can be performed by the same method as the mixing method in the step (c). For example, specifically, water may be used as the cleaning liquid.

The intermediate and the target product in the manufacturing method described above, for example, the intercalated product and the delaminated product may be dried by suction filtration, heat drying, freeze drying, vacuum drying, or the like.

In one aspect, the film may be binderless. In another aspect, the film may further comprise a resin in addition to the two-dimensional particles. Examples of the resin comprise an acrylic resin, a polyester resin, a polyamide resin, a polyimide resin, a polyamideimide resin, a polyolefin resin, a polycarbonate resin, a polyurethane resin, a polystyrene resin, a polyether resin, polylactic acid, and polyvinyl alcohol.

The film may further comprise other additives.

In one aspect, the electrode of the present disclosure may be formed only of the film. In another aspect, the electrode of the present disclosure may comprise the film and one or more selected from a substrate and a protective layer. The electrode may be in a solid state or in a flexible soft state.

When the electrode of the present embodiment comprises one or more selected from the substrate and the protective layer, the film and the one or more selected from the substrate and the protective layer may be in direct contact with each other.

The substrate may be, for example, an inorganic material such as ceramic or glass, or an organic material. Examples of the organic material comprise flexible organic materials, and specific examples thereof comprise a thermoplastic polyurethane elastomer (TPU), a PET film, and a polyimide film. The substrate may be a fiber material (for example, a sheet-shaped fiber material) such as paper or cloth.

The protective layer may be a layer covering at least a part or the entire of the film, and may be preferably a layer covering at least a part of the film. The protective layer may be made of an organic material, and specifically may be a resin such as an acrylic resin, a polyester resin, a polyamide resin, a polyimide resin, a polyamideimide resin, a polyolefin resin, a polycarbonate resin, a polyurethane resin, a polystyrene resin, a polyether resin, polylactic acid, or polyvinyl alcohol.

In the electrode of the present embodiment, the film may be exposed to the outside air so as to be in direct contact with an object to be measured, or may be covered with a substrate, a protective layer, or the like.

The method for manufacturing an electrode of the present disclosure comprises forming a film using the two-dimensional particles, and the two-dimensional particles comprise at least the intercalated product, and may comprise the intercalated product and the delaminated product and/or the delaminated cleaned product. In one aspect, the two-dimensional particles may preferably comprise the intercalated product and the delaminated product, and in another aspect, the two-dimensional particles may preferably comprise the intercalated product and the delaminated cleaned product.

Although it should not be construed as being limited to a specific theory, the intercalated product is considered to comprise metal cations, and adhesion between layers is moderately suppressed as compared with the etched product and the etched cleaned product. The intercalated product comprises more metal cations than those of the delaminated product and the delaminated cleaned product. Therefore, the usage of the two-dimensional particles comprising the intercalated product may result in a film comprising metal cations and having good film formability.

The two-dimensional particles used in the method for manufacturing an electrode of the present disclosure comprise the intercalated product, and the intercalated product is considered to be delaminated delamination to some extent due to a shear stress in a film forming process. Therefore, the film can be formed even when the two-dimensional particles do not comprise the delaminated product or the delaminated cleaned product.

The film can be formed, for example, by performing suction filtration of a mixed liquid comprising two-dimensional particles, a dispersion medium, and the resin to be used as necessary, or applying a mixed liquid comprising two-dimensional particles, a dispersion medium, and the resin to be used as necessary, and drying the dispersion medium once or twice or more.

For example, a supernatant comprising the two-dimensional particles (delaminated product) obtained by the delamination treatment and the intercalated product may be mixed to be used as the mixed liquid.

Examples of the method for applying the mixed liquid comprise a method for applying the mixed liquid by spraying. The spraying method may be, for example, an airless spraying method or an air spraying method, and specific examples thereof comprise a spraying method using a nozzle such as a one-fluid nozzle, a two-fluid nozzle, or an air brush.

As the dispersion medium, a medium similar to the dispersion medium can be used, and examples of the dispersion medium comprise water; and organic media such as N-methylpyrrolidone, N-methylformamide, N,N-dimethylformamide, methanol, ethanol, dimethylsulfoxide, ethylene glycol, and acetic acid.

The proportion of the intercalated product in the two-dimensional particles can be preferably 20 vol% to 100 vol%, more preferably 30 vol% to 100 vol% or less, and still more preferably 60 vol% to 100 vol% in 100 vol% of the two-dimensional particles. This may result in the formation of a film comprising a large amount of metal cations while maintaining film formability.

The total proportion of the intercalated product, the delaminated product, and the delaminated cleaned product in the two-dimensional particles can be preferably 0 vol% to 70 vol%, more preferably 0 vol% to 60 vol%, and still more preferably 0 vol% to 25 vol% in 100 vol% of the two-dimensional particles.

The intercalated product, the delaminated product, and the delaminated cleaned product can be manufactured by the manufacturing method described above, but are not limited thereto.

### (Application of Electrode)

The electrode of the present embodiment can be used for any suitable application. Examples thereof comprise a counter electrode and a reference electrode in electrochemical measurement, an electrode for an electrochemical capacitor, an electrode for a battery, a bioelectrode, an electrode for a sensor, an electrode for an antenna, and an electrical stimulation electrode. The electrode can also be used in applications where high moisture resistance (for example, reducing a decrease in initial conductivity and preventing oxidation) is required, such as electromagnetic shielding (EMI shielding). Details of these applications will be described below.

The electrode is not particularly limited, and may be, for example, an electrode for a capacitor, an electrode for a battery, a biological signal sensing electrode, an electrode for a sensor, an electrode for an antenna, and an electrical stimulation electrode and the like. According to the electrode of the present disclosure, it is possible to obtain a large-capacity capacitor and battery, a low-impedance biological signal sensing electrode, and a highly sensitive sensor and antenna even with a smaller volume (device occupied volume).

The capacitor may be an electrochemical capacitor. The electrochemical capacitor is a capacitor utilizing capacitance developed due to a physicochemical reaction between an electrode (electrode active material) and ions (electrolyte ions) in an electrolytic solution, and can be used as a device (power storage device) that stores electric energy. The battery may be a repeatedly chargeable and dischargeable chemical battery. The battery may be, for example, but not limited to, a lithium ion battery, a magnesium ion battery, a lithium sulfur battery, a sodium ion battery, or the like.

The biological signal sensing electrode is an electrode for acquiring a biological signal. The biological signal sensing electrode may be, for example, but not limited to, an electrode for measuring electroencephalogram (EEG), electrocardiogram (ECG), electromyogram (EMG), electrical impedance tomography (EIT).

The electrode for a sensor is an electrode for detecting a target substance, state, abnormality, or the like. The sensor may be, for example, but not limited to, a gas sensor, a biosensor (a chemical sensor utilizing a molecular recognition mechanism of biological origin), or the like.

The electrode for an antenna is an electrode for emitting an electromagnetic wave into a space and/or receiving an electromagnetic wave in a space. The antenna formed by the electrode for an antenna is not particularly limited, and examples thereof comprise antennas for mobile communication (antennas for so-called 3G, 4G and 5G) such as mobile phones, antennas for RFID, and antennas for near field communication (NFC).

The electrical stimulation electrode is an electrode for applying an electrical stimulation to a living body. The electrical stimulation can be applied to a living body, particularly a living tissue, for example, a spinal cord, a brain, a nerve tissue, a muscle tissue, or the like, but is not limited thereto.

Preferably, the electrode of the present embodiment can be used as a biological signal sensing electrode. Since the electrode of the present disclosure has low impedance, it is expected that the electrode can contribute to sensing with higher spatial resolution, and can be advantageously used for measuring biological signals such as electroencephalogram (EEG), electrocardiogram (ECG), electromyogram (EMG), and electrical impedance tomography (EIT).

Although the electrode and the two-dimensional particles used for the electrode in one embodiment of the present disclosure have been described in detail above, various modifications are possible. It should be noted that the electrode and the two-dimensional particles in the present disclosure may be manufactured by a method different from the manufacturing method in the above-described embodiment, and the manufacturing methods of the electrode and the two-dimensional particles in the present disclosure are not limited only to those providing the electrode and the two-dimensional particles in the above-described embodiment.

<1> An electrode comprising a film comprising two-dimensional particles,
   wherein the two-dimensional particles comprise at least a metal cation and one or a plurality of layers,
   the layer comprises:
      a layer body represented by a formula:

         MₘXₙ,
      wherein M is at least one Group 3, 4, 5, 6, or 7 metal and comprises at least a Ti atom, X is a carbon atom, a nitrogen atom, or a combination thereof, n is 1 or more and 4 or less, m is more than n and 5 or less; and
      a modifier or terminal T presents on a surface of the layer body,
      wherein T is at least one selected from the group consisting of a hydroxyl group, a fluorine atom, a chlorine atom, an oxygen atom, and a hydrogen atom,
      wherein the metal cation comprises a Li cation, and
      wherein a content of the Li cation in the two-dimensional particles is 5.4 mol or more with respect to 100 mol of the Ti atom.
<2> The electrode according to <1>, wherein the content of the Li cation in the two-dimensional particles is 5.4 mol or more and 9.7 mol or less with respect to 100 mol of the Ti atom.
<3> The electrode according to <1> or <2>, wherein the electrode is a biological signal sensing electrode.
<4> A method for manufacturing an electrode, the method comprising forming a film using two-dimensional particles,
   wherein the two-dimensional particles comprise
   at least an intercalated product manufactured by a manufacturing method comprising:
      (a) providing a precursor represented by a formula:

         MₘAXₙ

         wherein M is at least one Group 3, 4, 5, 6, or 7 metal and comprises at least a Ti atom, X is a carbon atom, a nitrogen atom, or a combination thereof, A is at least one Group 12, 13, 14, 15, or 16 element, n is 1 or more and 4 or less, and m is more than n and 5 or less;
   (b) removing at least a part of the A atom from the precursor using an etching solution to obtain an etched product;
   (c) cleaning the etched product to obtain an etched cleaned product; and
   (d) mixing the etched cleaned product with a metal compound comprising a metal cation to obtain an intercalated product in which the etched cleaned product is intercalated with the metal cation,
   wherein the metal cation comprises a Li cation.
<5> The method for manufacturing an electrode according to <4>,
   wherein the two-dimensional particles further comprise
   a delaminated product manufactured by a manufacturing method comprising
      (e) performing a delamination treatment of stirring the intercalated product to delaminate the intercalated product, thereby obtaining a delaminated product.
<6> The method for manufacturing an electrode according to <4> or <5>,
   wherein the two-dimensional particles further comprise
   a delaminated cleaned product manufactured by a manufacturing method comprising
   (e) stirring the intercalated product to obtain a delaminated product in which the intercalated product is delaminated, and
   (f) cleaning the delaminated product to obtain a delaminated cleaned product.
<7> The method for manufacturing an electrode according to any one of <4> to <6>,
   wherein the two-dimensional particles comprise at least the metal cation and one or a plurality of layers,
   the layer comprises:
   a layer body represented by a formula:

      MₙXₐ,
   wherein M, X, n, and m have the same meaning as described above; and
   a modifier or terminal T present on a surface of the layer body, wherein T is at least one selected from the group consisting of a hydroxyl group, a fluorine atom, a chlorine atom, an oxygen atom, and a hydrogen atom,
   the metal cation comprises a Li cation, and
   wherein a content of the Li cation in the two-dimensional particles is 5.4 mol or more with respect to 100 mol of the Ti atom.

### Examples

The present disclosure will be described more specifically with reference to the following Examples, but the present disclosure is not limited thereto.

### <Experimental Examples 1 to 6>

### <Preparation of Two-Dimensional Particles

In Experimental Examples 1 to 6, (1) preparation of a precursor (MAX), (2) etching of the precursor, (3) cleaning, (4) intercalation, (5) delamination and cleaning described in detail below were sequentially performed to prepare an intercalated product and a delaminated product.

### (1) Preparation of Precursor

TiC powder, Ti powder, and Al powder (all manufactured by Kojundo Chemical Laboratory Co., Ltd.) were placed in a ball mill containing zirconia balls at a molar ratio of 2 : 1 : 1 and mixed for 24 hours. The obtained mixed powder was calcined in an Ar atmosphere at 1350°C for 2 hours. The obtained calcined body (block) was pulverized with an end mill to a maximum size of 40 µm or less. Thereby, Ti₃AlC₂ particles were obtained as a precursor (MAX).

### (2) Etching of Precursor

Using the Ti₃AlC₂ particles (powder) prepared by the above method, etching was performed under the following etching conditions to obtain a solid-liquid mixture (slurry) comprising a solid component (etched product) derived from Ti₃AlC₂ powder.

### (Etching Conditions)

· Precursor: Ti₃AlC₂ (sieving with a mesh size of 45 µm)
· Etching solution composition:
   49%HF 6 mL
   H₂O 18 mL
   HCl (12 M) 36 mL
· Amount of precursor charged: 3.0 g
· Etching container: 100 mL I Boy
· Etching temperature: 35°C
· Etching time: 24h
· Stirrer rotation speed: 400 rpm

### (3) Cleaning

The slurry was divided into two portions, each of which was inserted into each of two 50 mL centrifuge tubes, centrifuged for 5 minutes under the condition of 3500 G using a centrifuge. Then, the supernatant was discarded. An operation of adding 35 mL of pure water to each centrifuge tube, and centrifuging again at 3500 G for 5 minutes to separate and remove the supernatant was repeated 11 times. After final centrifugation, the supernatant was discarded to obtain a clay of Ti₃C₂Tₛ (etched cleaned product, hereinafter also referred to as "two-dimensional particles (c)") and a water medium.

### (4) Intercalation

0.75 g of LiCl and 37.2 g of pure water were added to the clay of Ti₃C₂Tₛ (etched cleaned product) and a water medium, followed by stirring at 20°C or higher and 25°C or lower for 24 hours to perform intercalation using lithium ions as an intercalator, thereby obtaining an intercalated product (hereinafter also referred to as "two-dimensional particles (d)"). The detailed conditions of the intercalation are as follows.

### (Conditions of Intercalation)

· Ti₃C₂Tₛ-moisture medium clay (etched cleaned product): solid content 0.5 g
· Metal compound: LiCl 0.75 g
· Intercalation container: 100 mL I Boy
· Temperature: 20°C or higher and 25°C or lower (room temperature)
· Time: 24 hours
· Stirrer rotation speed: 700 rpm

### (5) Delamination and Cleaning

The slurry obtained by the intercalation was charged into a 50 mL centrifuge tube, and centrifuged for 5 minutes under the condition of 3,500 G using a centrifuge. Then, the supernatant was recovered to obtain a clay comprising two-dimensional particles (delaminated product). Furthermore, an operation of adding 35 mL of pure water, followed by stirring with a shaker for 15 minutes, then centrifuging the mixture at 3,500 G for 5 minutes, and collecting the supernatant as a liquid comprising single-layer MXene particles was repeated 4 times to obtain a supernatant comprising single-layer MXene particles. Furthermore, this supernatant was centrifuged using a centrifuge under the conditions of 4,300 G and 2 hours, and the supernatant was then discarded to obtain a clay comprising a delaminated cleaned product (hereinafter, also referred to as "two-dimensional particles (f)").

### [Preparation of Slurry]

The etched cleaned product, the intercalated product, and the delaminated cleaned product were placed in a 50 mL centrifuge tube so as to have ratios shown in Table 1, and pure water was added so that the ratio of the two-dimensional particles obtained by combining the intercalated product and the delaminated cleaned product was 1.5% by mass. Then, the mixture was stirred on a shaker for 15 minutes to obtain a slurry.

### [Preparation of Electrode]

The slurry was placed in a 25 mL syringe, and the syringe was set on a spray coater. Next, a 3 cm square polyimide substrate was set on a suction-equipped stage of a spray coater, and the slurry was applied 15 times to form a film, thereby obtaining a laminate comprising the polyimide substrate and an undried film disposed on the polyimide substrate. Conditions for spray coating were as follows.

### (Spray Coating Conditions)

· Atomization pressure: 0.5 MPa
· Distance between nozzle tip and substrate: 15 cm
· Liquid delivery amount: 5 mL/s
· Sweep speed: 150 mm/s
· Stage heater: 45°C

The laminate was dried using an atmospheric oven under the conditions of 80°C for 2 hours and then vacuum at 150°C overnight to form an electrode comprising the polyimide substrate and the film disposed on the polyimide substrate.

### (Method for Measuring Impedance)

The electrode was covered with a Kapton (registered trademark) tape, and an opening of 10 mmϕ was provided to serve as a working electrode. A beaker cell was assembled using a platinum electrode as a counter electrode and a silver-silver chloride electrode as a reference electrode. The counter electrode used had an area larger than that of the opening of the working electrode.

Next, a frequency was set to a range of 10 Hz, a voltage was set to an open circuit voltage or 1 mVrms to 20 mVrms with respect to the reference electrode, the number of plots was set to 71, and the number of N per plot was set to 1 to 10, and electrochemical measurement was performed in a potentiostat mode. As an electrochemical measurement apparatus, a VMP-300 high-performance electrochemical measurement system (16ch advanced model) manufactured by Bio-Logic Science Instruments was used.

### (Measurement of Li Atom Content by Inductively Coupled Plasma Atomic Emission Spectroscopy (ICP))

The electrode was made into a solution by an alkali melting method, and the obtained solution was measured by inductively coupled plasma atomic emission spectrometry (ICP-AES) to detect metal cations comprised in the two-dimensional particles. For the ICP-AES measurement, iCAP6300 (manufactured by Thermo Fisher Scientific) was used.

The results are shown in Table 1. Electrodes corresponding to Comparative Example of the present disclosure are denoted by symbols "*", and the other electrodes correspond to Examples of the present disclosure.

**[Table 1]**

| | Two-dimensional particles | | | Impedance | Li/Ti |
|---|---|---|---|---|---|
| | (c) | (d) | (f) | | |
| | (vol%) | (vol%) | (vol%) | (Ω) | (mol%) |
| 1 | - | 100 | - | 320.812 | 6.4 |
| 2 | - | 75 | 25 | 423.625 | 6.2 |
| 3 | - | 50 | 50 | 513.044 | 5.9 |
| 4* | 100 | - | - | 10000 | ND |
| 5* | 50 | - | 50 | 1322.84 | 3 |
| 6* | 0 | - | 100 | 541.816 | 5.3 |

Experimental Examples 1 to 3 are Examples of the present disclosure, and electrodes having low impedance were obtained. Experimental Examples 4 to 6 were examples in which the content of the metal cation was less than 5.4 mol with respect to 100 mol of a Ti atom, and the impedance was not sufficiently satisfactory.

### REFERENCE SIGNS LIST

1a, 1b Layer body (MₘXₙ layer)
3a, 5a, 3b, 5b Modifier or terminal T
7a, 7b MXene layer
10, 10a, 10b MXene particles (two-dimensional particles of layered material)

## Claims

1. An electrode comprising a film comprising two-dimensional particles,
wherein the two-dimensional particles comprise at least a metal cation and one or a plurality of layers,
the layer comprises:
a layer body represented by a formula:
MₘXₙ,
wherein M is at least one Group 3, 4, 5, 6, or 7 metal and comprises at least a Ti atom, X is a carbon atom, a nitrogen atom, or a combination thereof, n is 1 or more and 4 or less, m is more than n and 5 or less; and
a modifier or terminal T presents on a surface of the layer body, wherein T is at least one selected from the group consisting of a hydroxyl group, a fluorine atom, a chlorine atom, an oxygen atom, and a hydrogen atom,
wherein the metal cation comprises a Li cation, and
wherein a content of the Li cation in the two-dimensional particles is 5.4 mol or more with respect to 100 mol of the Ti atom.

2. The electrode according to claim 1, wherein the content of the Li cation in the two-dimensional particles is 5.4 mol or more and 9.7 mol or less with respect to 100 mol of the Ti atom.

3. The electrode according to claim 1 or 2, wherein the electrode is a biological signal sensing electrode.

4. A method for manufacturing an electrode, the method comprising forming a film using two-dimensional particles,
wherein the two-dimensional particles comprise
at least an intercalated product manufactured by a manufacturing method comprising:
(a) providing a precursor represented by a formula:
MₘAXₙ
wherein M is at least one Group 3, 4, 5, 6, or 7 metal and comprises at least a Ti atom, X is a carbon atom, a nitrogen atom, or a combination thereof, A is at least one Group 12, 13, 14, 15, or 16 element, n is 1 or more and 4 or less, and m is more than n and 5 or less;
(b) removing at least a part of the A atom from the precursor using an etching solution to obtain an etched product;
(c) cleaning the etched product to obtain an etched cleaned product; and
(d) mixing the etched cleaned product with a metal compound comprising a metal cation to obtain an intercalated product in which the etched cleaned product is intercalated with the metal cation,
wherein the metal cation comprises a Li cation.

5. The method for manufacturing an electrode according to claim 4,
wherein the two-dimensional particles further comprise
a delaminated product manufactured by a manufacturing method comprising (e) performing a delamination treatment of stirring the intercalated product to delaminate the intercalated product, thereby obtaining a delaminated product.

6. The method for manufacturing an electrode according to claim 4 or 5,
wherein the two-dimensional particles further comprise
a delaminated cleaned product manufactured by a manufacturing method comprising
(e) stirring the intercalated product to obtain a delaminated product in which the intercalated product is delaminated, and
(f) cleaning the delaminated product to obtain a delaminated cleaned product.

7. The method for manufacturing an electrode according to any one of claims 4 to 6,
wherein the two-dimensional particles comprise at least the metal cation and one or a plurality of layers,
the layer comprises:
a layer body represented by a formula:
MₘXₙ,
wherein M, X, n, and m have the same meaning as described above; and
a modifier or terminal T present on a surface of the layer body, wherein T is at least one selected from the group consisting of a hydroxyl group, a fluorine atom, a chlorine atom, an oxygen atom, and a hydrogen atom,
wherein the metal cation comprises a Li cation, and
wherein a content of the Li cation in the two-dimensional particles is 5.4 mol or more with respect to 100 mol of the Ti atom.
